Europäisches Patentamt

European Patent Office

Office européen des brevets

(11) Veröffentlichungsnummer: **0 244 716**
**A2**

## (12) EUROPÄISCHE PATENTANMELDUNG

(21) Anmeldenummer: 87106035.6

(22) Anmeldetag: 24.04.87

(51) Int. Cl.⁴: **G 01 N 21/59**, G 01 N 21/61, G 01 N 9/24

(30) Priorität: 03.05.86 DE 3615131

(43) Veröffentlichungstag der Anmeldung: 11.11.87 Patentblatt 87/46

(84) Benannte Vertragsstaaten: BE CH DE FR GB LI NL

(71) Anmelder: BROWN, BOVERI & CIE Aktiengesellschaft, Kallstadter Strasse 1, D-6800 Mannheim 31 (DE)

(72) Erfinder: Meinhold, Henner, Dr. Dipl.-Phys., Zwischen den Bächen 14a, D-6902 Sandhausen (DE)

(74) Vertreter: Rupprecht, Klaus, Dipl.-Ing. et al, c/o BROWN, BOVERI & CIE AG Postfach 10 03 51 Zentralbereich Patente, D-6800 Mannheim 1 (DE)

(54) Messvorrichtung.

(57) Die Erfindung bezieht sich auf eine Meßvorrichtung (1) zur Dichtebestimmung von Gasen in verschlossenen Kammern, insbesondere von druckgasisolierten Schaltanlagen. Aufgabe der Erfindung ist es, eine Meßvorrichtung (1) aufzuzeigen, die dauerhaft mit einer gasgefüllten Kammer verbunden werden kann. Die erfindungsgemässe Meßvorrichtung (1) weist ein Gehäuse (2) in Form eines Rhomboeders auf, das gasdicht verschlossen ist und zwei lichtdurchlässige Wände (2A, 2B) aufweist, die einander gegenüberliegend angeordnet sind. Der Innenraum des Gehäuses (2) steht mit der Kammer, die mit einem Gas gefüllt ist, dessen Dichte ermittelt soll, derart in Verbindung, daß der Innenraum des Gehäuses (2) ebenfalls mit dem Gas ausgefüllt wird, und zwar so, daß dieses die gleiche Dichte aufweist. Von einer Lichtquelle (3) werden parallel ausgerichtete Lichtstrahlen auf die erste durchlässige Wand (2A) geleitet. Die Lichtstrahlen durchlaufen das Gehäuse (2) und treten über die zweite lichtdurchlässige Wand (2B) aus dem Gehäuse aus. Sie treffen auf einen Strahlungsteiler (8) auf, und werden von diesem in zwei Strahlenbündel aufgeteilt, deren Intensität ermittelt wird. Das Verhältnis der ermittelten Intensitäten ist ein Maß für die Dichte des Gases, das sich in dem Gehäuse (2) befindet.

BROWN, BOVERI & CIE   AKTIENGESELLSCHAFT
Mannheim                          18.04.1986
Mp-Nr.562/86                      ZPT/P1-Kr/Kn


## Meßvorrichtung

Die Erfindung bezieht sich auf eine Meßvorrichtung gemäß
dem Oberbegriff des Patentanspruches 1.

Solche Meßvorrichtungen sind zur Dichtebestimmung von
Gasen vorgesehen, die in geschlossene Gehäuse eingefüllt
sind.

Schaltanlagen, die nach außenhin zu isolieren sind, werden oftmals druckgasisoliert ausgebildet. Die zur Isolierung vorgesehenen Kammern sind vorzugsweise mit reinem $SF_6$ gefüllt. Um Undichtigkeiten in den Kammern feststellen zu können, muß die Dichte des eingefüllten Gases
des öfteren überprüft werden. Es ist deshalb erforderlich, daß jede gasgefüllte Kammer mit einer solchen Meßvorrichtung ausgerüstet ist. Um die Kosten für solche
Meßvorrichtungen in Grenzen halten zu können, ist es
erforderlich, daß diese relativ preisgünstig hergestellt
werden können.

Der Erfindung liegt deshalb die Aufgabe zugrunde, eine
Meßvorrichtung für gasgefüllte Anlagen zu schaffen, die
einen relativ einfachen Aufbau aufweist.

Dieser Aufgabe wird erfindungsgemäß durch die kennzeichnenden Merkmale des Patentanspruches 1 gelöst.

Weitere erfindungswesentliche Merkmale sind in den Unteransprüchen gekennzeichnet.

Die Erfindung wird nachfolgend anhand einer Zeichnung näher erläutert.

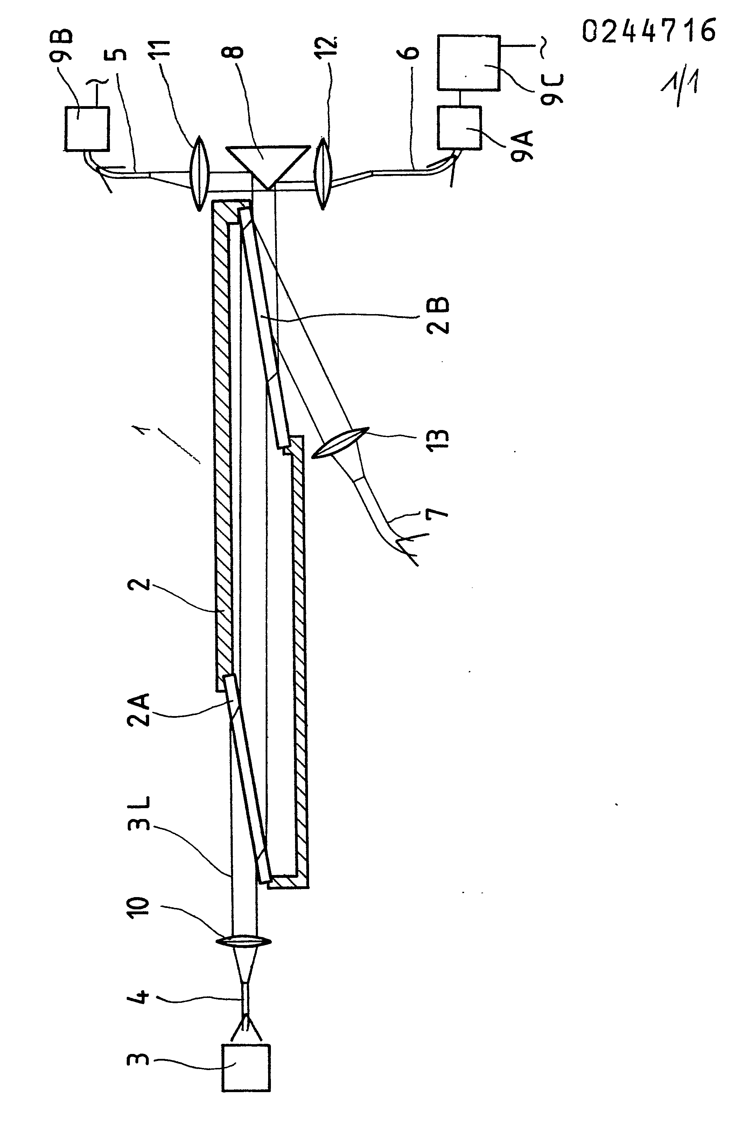

Figur 1 zeigt die erfindungsgemäße Meßvorrichtung 1, die im wesentlichen ein Gehäuse 2, eine Lichtquelle 3, vier Lichtleiter 4,5,6,7 einen Strahlungsteiler 8, sowie Linsen 10,11,12 und 13 aufweist. Das zur Meßvorrichtung 1 gehörende Gehäuse 2 ist mit einer Kammer (hier nicht dargestellt) verbunden, die mit einem Gas gefüllt ist, dessen Dichte kontinuierlich überwacht werden soll. Das Gehäuse 2, ist gasdicht ausgebildet. Es hat bei dem hier dargestellten Ausführungsbeispiel die Form eines Rhomboeders. Seine Wände sind gasdicht miteinander verbunden. Das Gehäuse 2 ist mit der oben erwähnten Kammer so verbunden, daß es mit dem in der Kammer enthaltenen Gas gefüllt wird. Die Verbindung ist so ausgebildet, daß das Gas in der Kammer und im Innenraum des Gehäuses 2 die gleiche Dichte aufweist. Zwei einander gegenüberliegende Wände 2A, 2B des Gehäuses 2 sind durchsichtig ausgebildet. Vorzugsweise sind sie aus Glas gefertigt. Sie schließen mit der Horizontalen einen Winkel von etwa 19° ein. Die übrigen begrenzenden Wände des Gehäuses 2 sind aus einem nicht durchsichtigen, jedoch ebenfalls gasdichten Material, beispielsweise einem Metall, gefertigt. Vor der ersten durchsichtigen Wand 2A des Gehäuses 2 ist eine Lichtquelle 3 installiert. Über einen Lichtleiter 4 wird das von der Lichtquelle 3 kommende Licht der Linse 10 zugeführt, die zwischen der Lichtquelle 3 und der Wand 2A angeordnet ist. Bei der Linse 10 handelt es sich um eine Sammellinse, die das von der Lichtquelle 3 kommende Licht parallel ausrichtet und auf die Oberfläche der Wand 2A leitet. Die Austrittsöffnug des Lichtleiters 4 ist im Brennpunkt der Linse 10 angeordnet. Vor der zweiten durchsichtigen Wand 2B ist ein Strahlungsteiler in Form eines Prismas 8 angeordnet. Dieses Prisma 8 hat die Aufgabe, das Licht, welches aus der durchsichtigen Wand 2B austritt in zwei Strahlenbündel aufzuteilen. Beidseitig des Prismas 8, insbesondere seiner Reflektionsflächen, welche das vom Gehäuse 2 kommende Licht teilen, ist je eine weitere Linse 11,12 angeordnet. Jede Linse 11,12 bildet den vom Prisma 8 kommenden Lichtstrahl auf den Lichtleiter 5,6 ab. Die Eintrittsöffnung des Lichtleiters 5,6 ist im Brennpunkt des Linse 11,12 angeordnet. Das an die Lichtleiter 5 und 6 übermittelte Licht wird an je ein Intensitätsmeßgerät 9A,9B weitergeleitet. Die ermittelten Intensitäten werden einer Auswerteeinrichtung 9C zugeführt, die den beiden Intensitätsmeßgeräten 9A,9B nachgeschaltet ist.

Um das Gerät in gewissen Zeitabständen neu einjustieren zu können, ist ein vierter Lichtleiter 7 vorgesehen, der im Bereich der zweiten durchsichtigen Wand 2B angeordnet ist. Über diesen Lichleiter 7 wird Licht von einer nicht näher dargestellten Lichtquelle der Linse 13 zugeführt, in deren Brennpunkt die Austrittsöffnung des Lichtleiters 7 angeordnet ist. Die Linse 13 richtet die Strahlen des vom Lichtleiter 7 kommenden Lichtes parallel aus und leitet sie auf die Oberfläche der Wand 2B. Der Lichtleiter 7 und die Linse 13 sind so angeordnet, daß die von der Linse 13 kommenden Lichtstrahlen so auf die durchlässige Wand 2B fallen, daß zwischen dieser Wand 2B und den Strahlen ein Winkel von 20° oder etwas mehr eingeschlossen wird. Die Strahlen, welche auf die Wand 2B fallen werden dort teilweise reflektiert und treffen auf den Strahlungsteiler 8 auf. Von diesem wer den sie in zwei Strahlungsbündel aufgeteilt und den
Lichtleitern 5 und 6 zugeführt.

Die Positionierung des Strahlungsteilers 8 erfolgt beim
Zusammenbau der Meßvorrichtung 1 in der Weise, daß zunächst Licht von der Lichtquelle 3, dessen Strahlen mit
Hilfe der Linse 10 parallel ausgerichtet werden, durch
das Gehäuse 2 hindurchgeschickt wird, wobei der Innenraum des Gehäuses 2 in diesem Fall evakuiert ist. Der
Strahlungsteiler 8 wird dann so vor der durchsichtigen
Wand 2B angeordnet, daß das aus dem Gehäuse 2 austretende Licht in zwei Strahlungsbündel aufgeteilt wird, welche die gleiche Intensität aufweisen.

Die Funktionsweise der erfindungsgemäßen Meßvorrichtung
1 wird nachfolgend beschrieben.

Um die Dichte eines Gases ermitteln zu können, das in
einer Schaltanlage (hier nicht dargestellt) enthalten
ist, wird die Kammer der Schaltanlage, die das Gas enthält, über eine Leitung (hier nicht dargestellt) mit dem
Gehäuse 2 verbunden, so daß der Innenraum des Gehäuses 2
mit dem selben Gas gefüllt werden kann, wie es in der
Kammer 2 enthalten ist, und zwar derart, daß das Gas in
dem Gehäuse die gleiche Dichte aufweist, wie das in der
Kammer enthaltene Gas. Die Verbindung zwischen dem Gehäuse 2 und einer solchen Kammer muß so ausgebildet
werden, daß selbst geringste Dichteschwankungen des Gases in der Kammer auch auf den Innenraum des Gehäuses 2
übertragen werden. Von der Lichtquelle 3 wird über den
Lichtleiter 4 und die Linse 10 parall ausgerichtetes
Licht auf die durchlässige Wand 2A des Gehäuses 2 geleitet. Das auf die Wand 2A auftreffende Licht dringt in
das Glas ein, und erfährt dabei eine Brechung. Das
Licht, welches aus der durchsichtigen Wand 2A austritt erfährt beim Übergang in den Innenraum des Gehäuses 2
eine weitere Brechung, deren Größe von der Dichte des in
dem Gehäuse 2 enthaltenen Gases abhängt. Eine zweite
Brechung erfährt das Licht beim Austritt aus dem Innenraum des Gehäuses, wenn es in die zweite durchsichtige
Wand 2B eintritt. Das Licht wird nochmals gebrochen,
wenn es aus der durchsichtigen Wand wieder in die mit
Luft gefüllte Umgebung austritt. Das von der zweiten
durchsichtigen Wand 2B kommende Licht trifft auf den
Strahlungsteiler 8 auf, und wird von diesem in zwei
Lichtbündel aufgeteilt, wobei jeweils ein Lichtbündel
einem Lichtleiter 5,6 zugeführt wird. Da der Strahlungsteiler so angeordnet ist, daß er das von der durchsichtigen Wand 2B kommende Licht nur dann in zwei
Strahlungsbündel aufteilt, die gleiche Intensität aufweisen, wenn der Innenraum des Gehäuses 2 luftleer ist,
erzeugt er in diesem Fall zwei Strahlungsbündel unterschiedlicher Intensität, da das von der durchsichtigen
Wand 2B kommende Licht nicht symmetrisch auf den Strahlungsteiler auftrifft. Die vom Strahlungsteiler 8 den
beiden Lichtleitern 5 und 6 zugeführten Strahlungsbündel
werden dem jeweils nachgeschalteten Intensitätsmeßgerät
9A,9B zugeführt. Das Verhältnis der Intensitäten der
beiden Strahlungsbündel ist ein Maß für die Dichte des
Gases, das sich im Inneren des Gehäuses 2 befindet.
Enthält das Innere des Gehäuses überhaupt kein Gas, so
erzeugt der Strahlungsteiler 8 zwei Strahlungsbündel
gleicher Intensität. Mit zunehmender Gasdichte wird die
Intensität des einen Bündels größer und die des anderen
kleiner, weil das durch das Gehäuse 2 hindurchgeleitete
Licht sowohl von den beiden durchsichtigen Wänden 2A und
2B als auch von dem im Inneren des Gehäuses 2 angeordneten Gas gebrochen wird. Um die erfindungsgemäße Meßvorrichtung 1 bei Bedarf eichen zu können, was insbesondere
bei zunehmendem Alter erforderlich wird, ist der vierte Lichtleiter 7 vorgesehen. Für die Eichung der Meßvorrichtung 1 wird die Lichtquelle 3 abgeschaltet und daß
von einer nicht dargestellten Lichtquelle kommende Licht
über den Lichtleiter 7 der Linse 13 zugeführt, die so
angeordnet ist, daß die von ihr parallel ausgerichteten
Lichtstrahlen vollständig auf die Außenfläche der Wand
2B auftreffen. Von dieser werden sie reflektiert, und
dem Strahlungsteiler 8 so zugeführt, daß dieser bei
ordnungsgemäßer Funktion der Meßvorrichtung 1 zwei
Strahlungsbündel mit gleicher Intensität erzeugt. Wird
bei der Überprüfung der Intensitäten der beiden über die
Lichtleiter 5 und 6 geleiteten Strahlenbündel festgestellt, daß ihre Intensitäten voneinander abweichen, so
ist dies ausschließlich auf Fehler in den Übertragungswegen zurückzuführen. Eine entsprechende Einjustierung
ist dann erforderlich.

Patentansprüche

1. Meßvorrichtung zur Dichtemessung von Gasen in gasdicht verschlossenen Kammern, insbesondere von druckgasisolierten Schaltanlagen, dadurch gekennzeichnet, daß ein mit der Kammer in Verbindung stehendes Gehäuse (2) mit wenigstens zwei einander gegenüberliegenden, lichtdurchlässigen Wänden (2A,2B) versehen ist, und daß die erste Wand (2A) gegenüber einer Lichtquelle (3) angeordnet und vor der zweiten Wand (2B) ein Strahlungsteiler (8) installiert ist.

2. Meßvorrichtung nach Anspruch 1, dadurch gekennzeichnet, daß das Gehäuse (2) als Rhomboeder ausgebildet ist.

3. Meßvorrichtung nach einem der Ansprüche 1 oder 2, dadurch gekennzeichnet, daß die Lichtquelle (3) ein Laser ist.

4. Meßvorrichtung nach einem der Ansprüche 1 bis 3, dadurch gekennzeichnet, daß der Strahlungsteiler ein Prisma (8) ist.

5. Meßvorrichtung nach einem der Ansprüche 1 bis 4, dadurch gekennzeichnet, daß vor der zweiten durchsichtigen Wand (2A) eine Justiereinrichtung (7,13) vorgesehen ist, die durch einen Lichtleiter (7) und eine nachgeschaltete Linse (13) gebildet ist, in deren Brennpunkt die Austrittsöffnung des Lichtleiters (7) angeordnet ist.

6. Meßvorrichtung nach Anspruch 1, dadurch gekennzeichnet, daß zwischen der Lichtquelle (3) und dem Gehäuse (2) eine Linse (10) angeordnet ist, in deren Brennpunkt die Austrittsöffnung eines mit der Lichtquelle (3) verbundenen Lichtleiters (4) installiert ist.

7. Meßvorrichtung nach einem der Ansprüche 1 bis 6,
dadurch gekennzeichnet, daß beidseitig des Strahlungsteilers (8) eine Linse (11,12) angeordnet ist, in deren
Brennpunkt der Eingang eines Lichtleiters (5,6) installiert ist.

0244716

1/1